Europäisches Patentamt

European Patent Office

Office européen des brevets

(19)

(11) Publication number: 0 487 090 A2

# EUROPEAN PATENT APPLICATION

(12)

(21) Application number: 91119883.6

(22) Date of filing: 21.11.91

(51) Int. Cl.5: C07D 201/04

(30) Priority: 21.11.90 JP 314203/90

(43) Date of publication of application:
27.05.92 Bulletin 92/22

(84) Designated Contracting States:
DE FR GB

(71) Applicant: UBE INDUSTRIES, LTD.
12-32, Nishihonmachi 1-chome
Ube-shi, Yamaguchi-ken 755(JP)

(72) Inventor: Inaba, Yukio, c/o Ube Research
Laboratory
Ube Industries, Ltd., 1978-5, Oaza Kogushi
Ube-shi, Yamaguchi-ken(JP)
Inventor: Kurokawa, Yasuhiro, c/o Ube
Research Laboratory
Ube Industries, Ltd., 1978-5, Oaza Kogushi
Ube-shi, Yamaguchi-ken(JP)
Inventor: Nawata, Yoshihiro, c/o Ube
Research Laboratory
Ube Industries, Ltd., 1978-5, Oaza Kogushi
Ube-shi, Yamaguchi-ken(JP)
Inventor: Kawai, Johji, c/o Ube Research
Laboratory
Ube Industries, Ltd., 1978-5, Oaza Kogushi
Ube-shi, Yamaguchi-ken(JP)

(74) Representative: Hansen, Bernd, Dr.
Dipl.-Chem. et al
Hoffmann, Eitle & Partner Patent- und
Rechtsanwälte Arabellastrasse 4 Postfach
81 04 20
W-8000 München 81(DE)

(54) Process for preparing caprolactam and laurolactam.

(57) There is disclosed a process for preparing caprolactam and laurolactam simultaneously by subjecting a mixture of cyclohexanone oxime and cyclododecanone oxime with a weight ratio of 40/60 to 70/30 to Beckmann rearrangement continuously in the presence of sulfuric acid and fuming sulfuric acid, under the conditions that amounts of the sulfuric acid and fuming sulfuric acid to be used in a rearrangement reaction solution are adjusted so as to satisfy the following formulae:

$$0.5 \text{ \% by weight} \leq \text{free } SO_3 \text{ concentration} \leq 4 \text{ \% by weight and} \quad 1.2 < m \leq 1.75$$

wherein m = (molar number of sulfuric acid + molar number of free $SO_3$)/(molar number of cyclohexanone oxime + molar number of cyclododecanone oxime)
and the rearrangement reaction is carried out at a temperature of 85 to 100 °C and a reaction time of 1 to 3 hours.

## BACKGROUND OF THE INVENTION

This invention relates to a process for preparing caprolactam and laurolactam simultaneously, more specifically to a process for preparing caprolactam and laurolactam simultaneously with high yields and high qualities by suitably adjusting conditions for converting a mixture of cyclohexanone oxime and cyclododecanone oxime into lactams in the presence of sulfuric acid and fuming sulfuric acid by continuously effecting Beckmann rearrangement.

Polyamide 12 has been attracted to attention since it has low water absorbing property so that it is excellent in dimensional stability and electric characteristics. Laurolactam which is a starting material therefor has been prepared as one of the industrial preparation methods by subjecting cyclododecanone oxime to Beckmann rearrangement. However, both of cyclododecanone oxime (melting point: 133 to 134 °C) and laurolactam (melting point: 152 to 153 °C) have high melting point so that a specific preparation technique is required for preparing it.

As one of the methods, there is a method in which caprolactam and laurolactam are simultaneously prepared. In this method, caprolactam and an intermediate thereof act as suitable solvent of laurolactam and an intermediate thereof so that the method has many advantages that a treatment at a relatively low temperature becomes easy. It is particularly preferred when a mixed polyamide is to be prepared.

As the methods for preparing caprolactam and laurolactam simultaneously, there have been known a method in which a mixture of cyclohexanone and cyclodedecanone is simultaneously oximated, the resulting mixture containing the produced cyclohexanone oxime or a salt thereof and cyclododecanone oxime or a salt thereof is subjected to Beckmann rearrangement in the presence of sulfuric acid or fuming sulfuric acid, then, a lactam mixture is obtained by neutralizing the above mixture with ammonia gas or aqueous ammonia, and the lactam mixture is extracted with an organic solvent which is immiscible with water or a lactam oil layer (hereinafter called to as "lactam oil") separated from an ammonium sulfate aqueous layer by phase separation of the lactam mixture is extracted with an organic solvent which is immiscible with water to obtain an extract containing lactam components, and further, the extract is reverse extracted with water to transfer caprolactam into the aqueous layer whereas dodecanelactam is retained in the organic solvent layer (so-called "colactamization method") (which method is described, for example, Japanese Patent Publication No. 7254/1971). Similar colactamization method has also been known as in the preparation of decalactam and caprolactam (for example, Japanese Patent Publication No. 10168/1971).

However, these methods are to be further improved in a yield of lactam and quality of the lactam obtained.

## SUMMARY OF THE INVENTION

An object of the present invention is to overcome the above problems and to provide a process for preparing caprolactam and laurolactam simultaneously with high yields and high qualities by suitably adjusting conditions for converting a mixture of cyclohexanone oxime and cyclododecanone oxime into lactams in the presence of sulfuric acid and fuming sulfuric acid by continuously effecting Beckmann rearrangement.

The present inventors have intensively studied to solve the above problems and by specifying the conditions of Beckmann rearrangement reaction to specific range, they have found that high quality lactams can be obtained with high yield whereby accomplished the present invention.

That is, the present invention is a process for preparing caprolactam and laurolactam simultaneously by subjecting a mixture of cyclohexanone oxime and cyclododecanone oxime with a weight ratio of 40/60 to 70/30 to Beckmann rearrangement continuously in the presence of sulfuric acid and fuming sulfuric acid, the improvement wherein amounts of the sulfuric acid and the fuming sulfuric acid to be used in a rearrangement reaction solution are adjusted so as to be a molar ratio m of a free $SO_3$ concentration and sulfuric acid to oximes satisfying the following formulae:

0.5 % by weight $\leq$ free $SO_3$ concentration $\leq$ 4 % by weight and     $1.2 < m \leq 1.75$

wherein m = (molar number of sulfuric acid + molar number of free $SO_3$)/(molar number of OX-6 + molar number of OX-12) where OX-6 represents cyclohexanone oxime and OX-12 represents cyclododecanone oxime,
and the rearrangement reaction is carried out under the following conditions:
rearrangement reaction a temperature:     85 to 100 °C
a reaction time:                          1 to 3 hours.

Also, after the rearrangement reaction, heat treatment of the rearrangement reaction mixture may be carried out under the following conditions.

heat treatment of the rearrangement reaction mixture

a temperature: 110 to 120 °C

a treatment time: 0.5 to 1.5 hours

## DESCRIPTION OF THE PREFERRED EMBODIMENTS

The present invention will be explained in detail below.

In the process for continuously preparing caprolactam and laurolactam by Beckmann rearrangement from a mixture of cyclohexanone oxime and cyclododecanone oxime, in view of melting points of mixtures of both starting materials and products and water contents of the starting materials, a weight ratio of cyclohexanone oxime and cyclododecanone oxime to be used is 40/60 to 70/30, preferably 40/60 to 60/40.

For increasing a yield of lactams and to obtain high quality lactams, it is important to prevent deterioration of the products during the reaction. For preventing deterioration, it is necessary to use sulfuric acid and free $SO_3$ with the molar numbers satisfying the following formula:

$$1.2 < m \leq 1.75$$

wherein m = (molar number of sulfuric acid + molar number of free $SO_3$)/(molar number of OX-6 + molar number of OX-12) and OX-6 and OX-12 have the same meanings as defined above. If m is not more than 1.2, quality of the products is lowered due to by-produced impurities. If m exceeds 1.75, deterioration of lactams becomes remarkable and the resulting lactam oil causes insoluble mud-like product in the subsequent procedures such as neutralization by ammonia gas or aqueous ammonia, extraction by an organic solvent and reverse extraction by water, whereby operations become difficult. Preferred range of m is $1.28 \leq m \leq 1.6$.

Also, a free $SO_3$ concentration in the rearrangement reaction solution is 0.5 % by weight $\leq$ free $SO_3$ concentration $\leq$ 4 % by weight, preferably 0.8 % by weight $\leq$ free $SO_3$ concentration $\leq$ 3 % by weight. If it is less than 0.5 % by weight, quality of the product is lowered, while it exceeds 4 % by weight, deterioration of caprolactam and laurolactam becomes remarkable and phase separation of a lactam oil and ammonium sulfate or aqueous ammonium sulfate become difficult in the subsequent neutralization by ammonia gas or aqueous ammonia.

The temperature at the rearrangement reaction is 85 to 100 °C, preferably 90 to 95 °C, and the reaction time is 1 to 3 hours, preferably about 2 hours.

A heat treatment temperature of the rearrangement reaction solution is 110 to 120 °C, preferably 115 to 120 °C and a time thereof is 0.5 to 1.5 hours, preferably about one hour. This heat treatment is carried out at a relatively high temperature so that, in order to prevent decomposition of the formed lactams, it is particularly desired to avoid heating the resulting product for a long period of time and make the molar ratio m of used sulfuric acid 1.6 or less and the free $SO_3$ concentration 0.8 to 2.5 % by weight, respectively. By effecting the heat treatment of the rearrangement reaction solution, quality of caprolactam becomes particularly excellent.

The product obtained by Beckmann rearrangement as mentioned above can be processed according to the method well known in the art. For example, the rearrangement reaction solution is neutralized by using aqueous ammonia or sodium hydroxide to obtain lactam mixture, and the lactam mixture is extracted with an organic solvent which is immiscible with water or a lactam oil separated from a sulfate aqueous solution phase by phase separation of the lactam mixture is extracted with an organic solvent which is immiscible with water to obtain an extract containing lactam components. Then, the extract is reverse extracted with water to transfer caprolactam to aqueous phase while remaining laurolactam in the organic solvent phase. If necessary, evaporation processing may be carried out.

## EXAMPLES

In the following, the present invention will be explained in more detail by referring to Examples, but the scope of the present invention is not limited by these.

Example 1 (m: 1.58, free $SO_3$ concentration: 1 % by weight)

An oxime mixture comprising 49 % by weight of cyclohexanone oxime, 49 % by weight of

EP 0 487 090 A2

cyclododecanone oxime and 2 % by weight of water and a prepared fuming sulfuric acid comprising 44.2 % by weight of 98 % sulfuric acid and 55.8 % by weight of 26 % fuming sulfuric acid were fed continuously with a weight ratio of 49 : 51 into a reactor made of glass with a jacket having a volume of 1 liter and equipped with a stirrer, a condenser and an overflow line while stirring so as to become the residence time of the solution being 2 hours. In the jacket, warm water at about 60 °C was fed and an amount of the warm water was controlled so as to become the temperature in the reactor being 90 to 95 °C.

After 8 hours when the reaction becomes stationary state, a rearrangement reaction solution drawn from the overflow line was accepted in a glass vessel for 2 hours. The resulting rearrangement reaction solution (1420 g) was further charged into a reaction vessel made of glass having a volume of 5 liter and equipped with a stirrer and a condenser, and heat treatment of the rearrangement reaction solution was carried out at 120 °C for one hour while stirring.

Next, 1720 g of 40 % by weight aqueous ammonium sulfate solution was charged into a vessel made of glass with a jacket having a volume of 5 liter and equipped with a pH meter, a stirrer and a condenser, and elevated to 100 °C by passing steam in the jacket. Then, in the pH range of 5.4 to 5.6, 1200 g of the rearrangement reaction solution which is a part of the solution subjected to heat treatment and 1560 g of a 14 % aqueous ammonia were simultaneously added dropwise to the ammonium sulfate solution over about 2 hours. The resulting neutralized solution was allowed to stand at 95 to 100 °C to separate 640 g of a lactam oil phase comprising caprolactam and laurolactam and 3830 g of 40 % by weight aqueous ammonium sulfate phase.

Yields based on oxime from the rearrangement reaction part to neutralization and separation were each 98 % for caprolactam and laurolactam, respectively. To the separated aqueous ammonium sulfate phase was added 2000 g of toluene, and extraction was carried out at the temperature range of 60 to 65 °C for 15 minutes to recover caprolactam in the aqueous ammonium sulfate phase into the toluene phase. This toluene phase was divided into two, and the previous lactam oil phase was extracted with one of the above toluene phase at 60 to 65 °C while stirring. Separated aqueous phase which is extraction residue was similarly extracted with the remaining toluene phase while stirring. The mixture of the above two toluene extracts contained 10 % by weight of caprolactam and 11 % by weight of laurolactam.

|  | Lactam oil phase | 40 % by weight aqueous ammonium sulfate |
| --- | --- | --- |
| Caprolactam | 40 % by weight | 0.8 % by weight |
| Laurolactam | 44 % by weight | 0.0 % by weight |

The toluene solution of caprolactam and laurolactam obtained was extracted with 4000 g of water at 60 °C to extract caprolactam into aqueous phase. The resulting aqueous solution of caprolactam was processed with a cation exchange resin (Amberlite 200C, trade name, available from Organo Co.) and an anion exchange resin (Amberlite IRA-900, trade name, available from Organo Co.), and crude caprolactam obtained by condensation was further subjected to vacuum distillation by adding 0.16 % of NaOH based on pure caprolactam to obtain caprolactam product. Qualities of the resulting caprolactam are as follows.

0.1N potassium permanganate consumed amount:     1.80 cc/kg

UV-ray transmittance (290 nm, 50 % solution) :     98.0 %

0.1N potassium permanganate consumed amount was calculated by dissolving 100 g of caprolactam in 250 ml of 8 mole/liter concentration sulfuric acid and titrating with 0.1N potassium permanganate aqueous solution.

Also, UV-ray transmittance is a transmittance of UV-ray at a wavelength of 290 nm to a 50 % aqueous solution of caprolactam, and it decreases due to the presence of impurities such as an aromatic amine or an azo compound.

Example 2 (m: 1.71, free $SO_3$ concentration: 2 % by weight)

The reaction was carried out in the same manner as in Example 1 except for feeding the oxime mixture as in Example 1 and a prepared fuming sulfuric acid comprising 40.7 % by weight of 98 % sulfuric acid and 59.3 % by weight of 26 % fuming sulfuric acid with a weight ratio of 47.4 : 52.6 for the rearrangement reaction.

Yields based on oxime from the rearrangement reaction to neutralization and separation were 98 % for caprolactam and 97 % for laurolactam, respectively.

4

Example 3 (m: 1.58, free SO$_3$ concentration: 1 % by weight, no heat treatment for the reaction mixture)

The reaction was carried out in the same manner as in Example 1 except for effecting heat treatment of the rearrangement reaction solution. Yields based on oxime were each 98 % for caprolactam and laurolactam, respectively.

Further, in the same manner as in Example 1, caprolactam was extracted with water from the toluene solution and ion exchange resin treatment, condensation and distillation were carried out to obtain caprolactam product. Qualities of the resulting caprolactam are as follows.

0.1N potassium permanganate consumed amount:     1.65 cc/kg
UV-ray transmittance (290 nm, 50 % solution) :       91.6 %

Comparative example 1 (m: 2.0, free SO$_3$ concentration: 4 % by weight, rearrangement reaction temperature: 100 °C)

The reaction was carried out in the same manner as in Example 1 except for feeding the oxime mixture as in Example 1 and a prepared fuming sulfuric acid comprising 34.3 % by weight of 98 % sulfuric acid and 65.7 % by weight of 26 % fuming sulfuric acid with a weight ratio of 43.8 : 56.2 for the rearrangement reaction and carrying out the rearrangement reaction at an inner vessel temperature of 98 to 103 °C. During neutralization using aqueous ammonia, a large amount of mud-like insolubles occurred and phase separation of the lactam oil phase and aqueous ammonium sulfate can be difficultly done.

Yields based on oxime from the rearrangement reaction to neutralization were 95 % for caprolactam and 67 % for laurolactam, respectively.

Example 4 (m: 1.39, free SO$_3$ concentration: 1 % by weight)

The reaction was carried out in the same manner as in Example 1 except for feeding an oxime mixture comprising 57.7 % by weight of cyclohexanone oxime, 38.5 % by weight of cyclododecanone oxime and 3.8 % by weight of water, and a prepared fuming sulfuric acid comprising 15.6 % by weight of 98 % sulfuric acid and 84.4 % by weight of 26 % fuming sulfuric acid with a weight ratio of 52.2 : 47.8 for the rearrangement reaction.

Yields based on oxime were 98.6 % for caprolactam and 95.7 % for laurolactam, respectively.

Further, in the same manner as in Example 1, caprolactam was extracted with water from the toluene solution and ion exchange resin treatment, condensation and distillation were carried out to obtain caprolactam product. Qualities of the resulting caprolactam are as follows.

0.1N potassium permanganate consumed amount:     2.4 cc/kg
UV-ray transmittance (290 nm, 50 % solution) :       91.6 %

Example 5 (m: 1.42, free SO$_3$ concentration: 2 % by weight)

The reaction was carried out in the same manner as in Example 1 except for feeding an oxime mixture comprising 67.2 % by weight of cyclohexanone oxime, 28.8 % by weight of cyclododecanone oxime and 4.0 % by weight of water, and a prepared fuming sulfuric acid comprising 11.0 % by weight of 98 % sulfuric acid and 89.0 % by weight of 26 % fuming sulfuric acid with a weight ratio of 50.5 : 49.5 for the rearrangement reaction.

Yields based on oxime were 98.1 % for caprolactam and 96.2 % for laurolactam, respectively.

Further, in the same manner as in Example 1, caprolactam was extracted with water from the toluene solution and ion exchange resin treatment, condensation and distillation were carried out to obtain caprolactam product. Qualities of the resulting caprolactam are as follows.

0.1N potassium permanganate consumed amount:     1.7 cc/kg
UV-ray transmittance (290 nm, 50 % solution) :       95.1 %

Comparative example 2 (m: 1.81, free SO$_3$ concentration: 4 % by weight, rearrangement reaction temperature: 100 °C)

The reaction was carried out in the same manner as in Example 1 except for feeding an oxime mixture comprising 57.7 % by weight of cyclohexanone oxime, 38.5 % by weight of cyclododecanone oxime and 3.8 % by weight of water, and a prepared fuming sulfuric acid comprising 12.8 % by weight of 98 % sulfuric acid and 87.2 % by weight of 26 % fuming sulfuric acid with a weight ratio of 45.6 : 54.4 for the rearrangement reaction and carrying out the rearrangement reaction at an inner vessel temperature of 98 to 103 °C. During neutralization using ammonia, mud-like insolubles occurred as in Comparative example 1.

Yields based on oxime from the rearrangement reaction to neutralization reaction were 93 % for caprolactam and 71 % for laurolactam, respectively.

Comparative example 3 (m: 1.90, free $SO_3$ concentration: 4.5 % by weight, no heat treatment of the reaction mixture)

The reaction was carried out in the same manner as in Example 1 except for feeding an oxime mixture comprising 49.0 % by weight of cyclohexanone oxime, 49.0 % by weight of cyclododecanone oxime and 2.0 % by weight of water, and a prepared fuming sulfuric acid comprising 30.3 % by weight of 98 % sulfuric acid and 69.7 % by weight of 26 % fuming sulfuric acid with a weight ratio of 45 : 55 for the rearrangement reaction and effecting no heat treatment of the reaction mixture. During neutralization using ammonia, mud-like insolubles occurred as in Comparative example 1 and a large amount of insolubles was migrated into aqueous ammonium sulfate.

Yields based on oxime from the rearrangement reaction to neutralization reaction were 83 % for caprolactam and 66 % for laurolactam, respectively.

Comparative example 4 (m: 1.58, free $SO_3$ concentration: 4.6 % by weight)

The reaction was carried out in the same manner as in Example 1 except for feeding an oxime mixture comprising 49.0 % by weight of cyclohexanone oxime, 49.0 % by weight of cyclododecanone oxime and 2.0 % by weight of water, and a prepared fuming sulfuric acid comprising 23.2 % by weight of 98 % sulfuric acid and 76.8 % by weight of 26 % fuming sulfuric acid with a weight ratio of 49.6 : 50.4 for the rearrangement reaction. During neutralization using 14 % aqueous ammonia, mud-like insolubles occurred as in Comparative example 1 and a large amount of insolubles was migrated into aqueous ammonium sulfate.

Yields based on oxime from the rearrangement reaction to neutralization reaction were 94 % for caprolactam and 72 % for laurolactam, respectively.

Comparative example 5 (m: 1.59, free $SO_3$ concentration: 0.3 % by weight)

The reaction was carried out in the same manner as in Example 1 except for feeding an oxime mixture comprising 49.0 % by weight of cyclohexanone oxime, 49.0 % by weight of cyclododecanone oxime and 2.0 % by weight of water, and a prepared fuming sulfuric acid comprising 48.3 % by weight of 98 % sulfuric acid and 51.7 % by weight of 26 % fuming sulfuric acid with a weight ratio of 49 : 51 for the rearrangement reaction.

Yields based on oxime from the rearrangement reaction to neutralization reaction were 99 % for caprolactam and 98 % for laurolactam, respectively.

Further, in the same manner as in Example 1, caprolactam was extracted with water from the toluene solution and ion exchange resin treatment, condensation and distillation were carried out to obtain caprolactam product. Qualities of the resulting caprolactam are as follows, which are worse in qualities than those of Example 1.

0.1N potassium permanganate consumed amount:     5.0 cc/kg

UV-ray transmittance (290 nm, 50 % solution) :     65.4 %

As described above, in Examples in which Beckmann rearrangement is carried out under the conditions of the present invention, yields and qualities of the resulting lactams are clearly excellent than those of Comparative examples.

## Claims

1. A process for preparing caprolactam and laurolactam simultaneously by subjecting a mixture of cyclohexanone oxime and cyclododecanone oxime with a weight ratio of 40/60 to 70/30 to Beckmann rearrangement continuously in the presence of sulfuric acid and fuming sulfuric acid, the improvement wherein amounts of the sulfuric acid and the fuming sulfuric acid to be used in a rearrangement reaction solution are adjusted so as to satisfy the following formulae:

0.5 % by weight $\leq$ free $SO_3$ concentration $\leq$ 4 % by weight and     $1.2 < m \leq 1.75$

wherein m = (molar number of sulfuric acid + molar number of free $SO_3$)/(molar number of OX-6 + molar number of OX-12) where OX-6 represents cyclohexanone oxime and OX-12 represents cyclododecanone oxime,

and the rearrangement reaction is carried out at a temperature of 85 to 100 °C and a reaction time of 1 to 3 hours.

2. The process according to Claim 1, wherein the weight ratio of cyclohexanone oxime and cyclododecanone oxime is 40/60 to 60/40.

3. The process according to Claim 1, wherein m is a number satisfying the following formula:

$$1.28 \leq m \leq 1.6.$$

4. The process according to Claim 1, wherein the reaction temperature is 90 to 95 °C.

5. The process according to Claim 1, wherein the reaction time is about 2 hours.

6. The process according to Claim 2, wherein the reaction is carried out under the conditions of $1.28 \leq m \leq 1.6$, the reaction temperature of 90 to 95 °C and the reaction time of about 2 hours.

7. The process according to Claim 6, wherein the reaction is carried out under the conditions of m being 1.6 or less and the free $SO_3$ concentration of 0.8 to 2.5 % by weight.

8. The process according to Claim 1, wherein heat treatment is further carried out after the rearrangement reaction under the conditions of a temperature of 110 to 120 °C for 0.5 to 1.5 hours.

9. The process according to Claim 8, wherein the heat treatment is carried out at a temperature of 115 to 120 °C for about one hour.